(19) Europäisches Patentamt
European Patent Office
Office européen des brevets

(11) Publication number: **0 093 770 B1**

(12) **EUROPEAN PATENT SPECIFICATION published in accordance with Art. 158(3) EPC**

(45) Date of publication of patent specification: **19.06.91**

(51) Int. Cl.[5]: **A61K 7/06**, A61K 31/505

(21) Application number: **83900123.7**

(22) Date of filing: **08.11.82**

(86) International application number: **PCT/US82/01593**

(87) International publication number: **WO 83/02558 (04.08.83 83/18)**

The file contains technical information submitted after the application was filed and not included in this specification

(54) **THE USE OF RETINOIDS AND MINOXIDIL (2,4-DIAMINO-6-PIPERIDINO-PYRIMIDINE-3-OXIDE) TO INCREASE THE RATE OF GROWTH OF HUMAN SCALP HAIR AND TO TREAT CERTAIN TYPES OF ALOPECIAS.**

(30) Priority: **09.11.81 US 318607**

(43) Date of publication of application: **16.11.83 Bulletin 83/46**

(45) Publication of the grant of the patent: **19.06.91 Bulletin 91/25**

(84) Designated Contracting States: **AT BE CH DE FR GB LI LU NL SE**

(56) References cited:
**WO-A-82/02833** **FR-A- 2 293 193**
**GB-A- 1 466 062** **US-A- 3 461 461**
**US-A- 4 126 698** **US-A- 4 139 619**

**Eur. Journal of Medical Chemistry, 1980, vol. 15, page 9-15**

(73) Proprietor: **BAZZANO, Gail S
4506 Avron Blvd.
Metairie, LA 70002(US)**

(72) Inventor: **BAZZANO, Gail S
4506 Avron Blvd.
Metairie, LA 70002(US)**

(74) Representative: **Casalonga, Axel et al
BUREAU D.A. CASALONGA - JOSSE Morassistrasse 8
W-8000 München 5(DE)**

**EP 0 093 770 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Annals of the N.Y. Academy of Sciences, vol. 359, 1981, page 1-13

Cancer Research, vol. 40, 1980, pages 3413-3425

## Description

This invention relates to compositions useful for increasing the rate of growth of hair and for treating various types of alopecias, comprising a retinoid and a 6-amino 4-(substituted amino) 1,2-dihydro 1-hydroxy 2-imino pyrimidine compound.

A normal characteristic of human hair growth in most individual is that it diminishes with age. Both the rate of growth of the hair and the length of the growing cycle are reduced. This condition is common to all individuals with rare exception and must be differentiated from true male pattern alopecia which is a distinct clinical entity associated in certain individuals with the production of the male sex hormone, testosterone and its derivatives, particularly dihydrotestosterone.

Many factors may influence the rate of hair growth including race, sex, age, region, season of the year, nutrition and hormones (Myers and Hamilton, 1951, Hamilton, 1958, Yano, 1936, Maeda, 1938, Troter, 1923, Pinkus, 1924, and Ono,1963). In the past, many attempts have been made to alter the course of male pattern alopecia without success.

US-A-4.139.618 discloses the use of minoxidil alone as stimulating the growth of mammalian hair.

This invention concerns composition useful for increasing the rate of growth of hair, and for treating various type of alopecias comprising a retinoid and a 6-amino 4- (substituted amino) 1,2-dihydro 1-hydroxy 2-imino pyrimidine compound of formula :

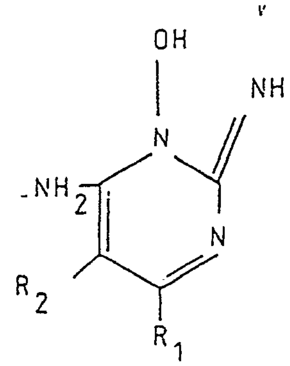

wherein $R_1$ is a moiety selected from moieties of the formula

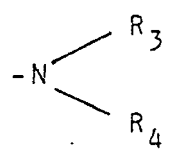

wherein $R_3$ and $R_4$ are selected from hydrogen, lower alkyl, lower alkenyl, lower aralkyl, and lower cycloalkyl, and taken together $R_3$ and $R_4$ may be a heterocyclic moiety selected from aziridinyl, azetidinyl, pyrrolidinyl, piperidino, hexahydroazepinyl, heptamethylenimino, octamethylenimino; morpholino, and 4-(lower-alkyl)piperazinyl, each of said hetero cyclic moieties having attached as substituents on the carbon atoms 0-3 lower alkyl groups, hydroxy or alkoxy wherein $R_2$ is selected from hydrogen, lower alkyl, lower alkenyl, lower alkoxyalkyl, lower cycloalkyl, lower aryl, lower arylalkyl, lower alkylaryle lower alkylarylalkyl, lower alkoxyarylalkyl, and lower haloarylalkyl and the pharmacologically acceptable acid addition salts thereof, in an amount producing a synergistic effect.

The present inventor has applied for US and PCT patents 235,169 and PCT-US 81 00338 respectively on February 17, 1981 and on March 30, 1981 on the use of retinoids in the promotion of hair growth. All-trans retinoic acid has been shown to cause elevated DNA synthesis in keratinocytes in cell culture. All-trans retinoic acid can also be shown to increase the turn over time of epidermal cells in cell culture experiments as well as in vivo experiments with human subjects. The present inventor has discovered that the cells of the hair follicle including the papillae can be stimulated by retinoids including all-trans retinoic acid. When tested experimentally, the retinoids caused the cells of the hair follicle to incorporate more tritiated thymidine into DNA and to reproduce at a more rapid rate than untreated cells from other hair

follicles. This stimulation by the retinoid compounds ultimately causes the entire hair follicle to become more activated and the mitotic index as measured by thymidine-$H^3$ incorporation into DNA to rise. Therefore, the individual scalp hair can be shown to grow at an increased rate and the anagen phase is prolonged.

A major problem in influencing alopecia is to revascularize the area of alopecia and initiate the primary new hair growth. All-trans retinoic acid and its derivatives and the other retinoid compounds have been shown to give excellent percutaneous absorption and to be very active on the keratinizing cells of the skin, including the hair follicle, however, it is difficult for retinoids alone to revascularize the area of the pilosebaceous apparatus.

Studies have shown that minoxidil, a potent antihypertensive medication and peripheral vasodilator can increase the rate of hair growth on the body when taken systemically, particularly in areas of the limbs and facial areas, primarily due to vasodilatory properties. Further studies have suggested that minoxidil may be effective in initiating and promoting vellus hair growth on the scalp in individuals with alopecia, however, minoxidil is not able to sustain the growth of terminal hairs from vellus hairs on the scalp; and in the majority of subjects with alopecia, even pronounced vellus hair growth on the scalp is not initiated or sustained by the topical application of minoxidil nor by the systemic administration.

The mechanism by which minoxidil exerts its effect is believed to be revascularization and by its vasodilatory properties, however, vasodilation alone is not sufficient stimulus for hair growth, particularly in an area affected by alopecia. The present inventor has applied for patent PCT-US-81-00338 on March 12, 1981, and has shown that certain retinoids can increase the rate of hair growth in both males and females and can prolong the anagen phase of the hair cycle thereby converting vellus to terminal hair. The mechanism of action of the retinoid compounds is believed to be through the initiation and activation of increased cell differentiation, i.e., compounds which by themselves can initiate the differentiation of cells of the pilosebaceous apparatus which eventually form the hair follicle and become terminal hairs.

Because of the advanced state of scalp thinning and atrophy of the pilary portion of the pilosebaceous apparatus, it is difficult to initiate hair growth from areas of advanced male pattern alopecia. Certain of the retinoid compounds sustain and promote hair growth in areas where some hair is present to some extent. However, in very advanced cases of complete or total male pattern alopecia, certain retinoids may not produce sufficient new hair growth.

The present application is for the use of certain retinoid compounds with minoxidil. The stimulatory actions of both compounds can promote each other i.e., synergism, retinoids can initiate cell growth and differentiation (not initiated by minoxidil) and minoxidil can promote the vasodilatory action not readily obtained with the retinoids. While neither compound alone can have profound effects on advanced alopecias, in combination the compounds are very effective as promoters of new hair growth in areas of alopecia.

The net result of application of minoxidil and retinoids is initiating and production of new hair growth and conversion of vellus to terminal hair, i.e., the increase in size from a vellus to a terminal hair and the continued and more prolonged maintenance of the hair in the anagen phase.

As noted previously, this effect is obtained not merely as the addition of the effects of two compounds but as synergism,i.e. the combination of these substances in the present invention produces an effect which cannot be produced by either compound separately under conditions of its use and, therefore, represents a major advance in the treatment of alopecia.

As used herein, the term retinoid denotes retinol, retinal, retinyl esters as well as retinoic acid and its esters derivatives and normal metabolites. The terminal group may be oxidized, reduced, esterified, etc. The alkali metal (sodium, potassium, etc.) and alkaline earth metal (magnesium calcium, etc.) salts are also included herein.

The retinoid used according to invention is an ester, an ether or a salt of a retinoid acid or a stereoisomer of vitamin A acid or vitamin $A_2$ acid, 5,6-epoxy vitamin A acid, dehydro vitamin A acid, anhydro vitamin A acid or the aldehyde, alcohols, esters, ethers thereof, or a naturally occuring metabolite of vitamin A or vitamin A acid. The metabolites are 4-hydroxy retinoic acid, 4-keto retinoic acid, 4-oxo retinoic acid, 5,8-oxy retinoic acid or stereoisomers thereof, aldehyde and esters thereof.

Retinoids useful according to the invention can be defined by formula

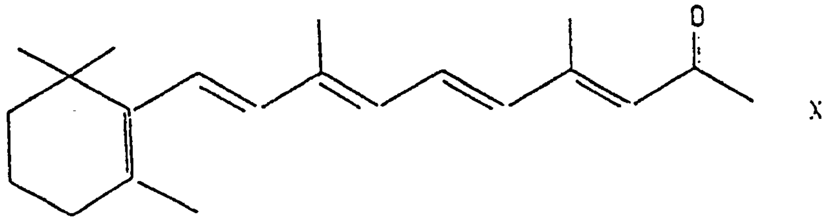

wherein X has the meanings
$-OCH_2CONH_2$; mixed $-OCH_2CH(OH)CH_3$ and
$-OCH(CH_3)CH_2OH$ :$-OCH_2CH_2OH$ :

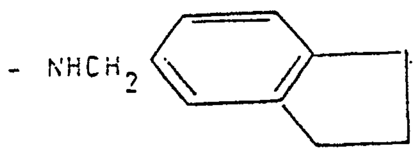

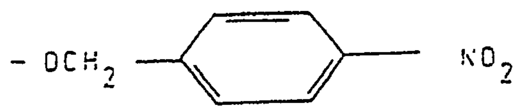

$-OCH_2C_6H_5$, or 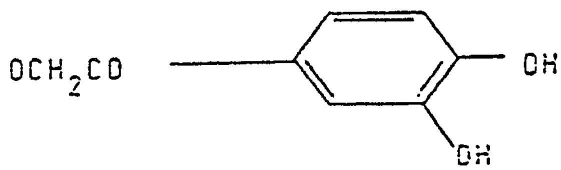

The retinoids are preferably selected from all-trans retinoic acid, all-trans retinoic acetate, retinaldehyde, 13-cis retinoic acid, retinoic acid ethylamide, retinoic acid 2-hydroxyethyl amide, retinoic acid p-hydroxyphenylamide, axerophthene, retinal, retinol, retinyl methylether, β-all-trans retinoic acid, 7,8-dehydro analog of retinoic acid, 5,6-epoxy analog of retinoic acid, retinyl methylthioether, 4-oxo analog of retinoic acid, N-methyl dimethyl dioxolan retinamide, retinyl n-butyl ether, N-(O-carboxyphenyl) retinamide, retinyl tert-butyl ether, retinylacetate, N-(p-carboxyphenyl) retinamide, N-benzoyl retinylamide, retinyl palmitate, retinylidene ethylcyano acetate.

Other retinoids correspond to compounds of formula

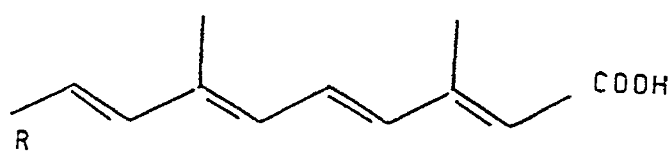

R having the meanings :

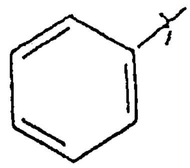

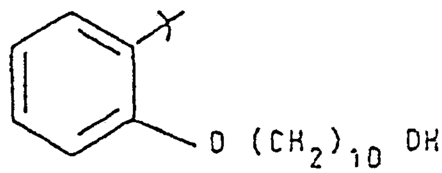

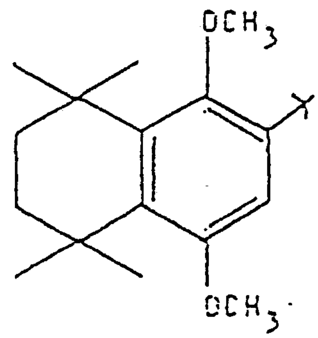

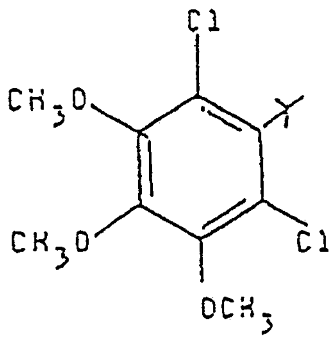

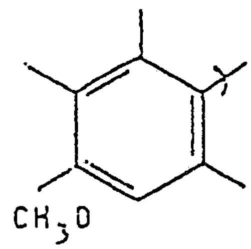

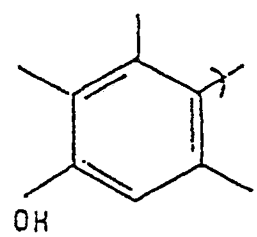

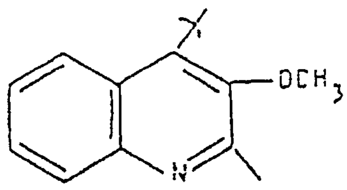

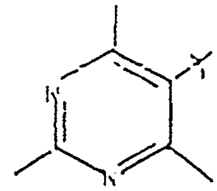

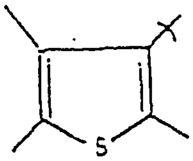

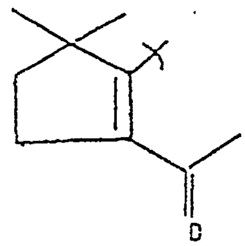

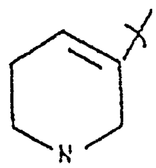

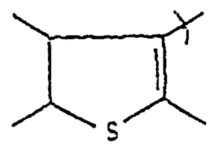

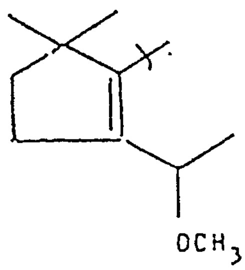

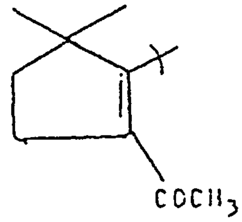

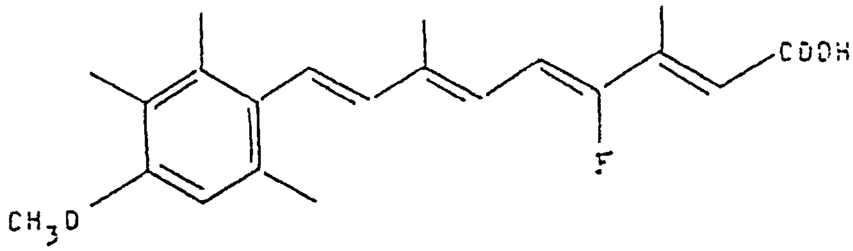

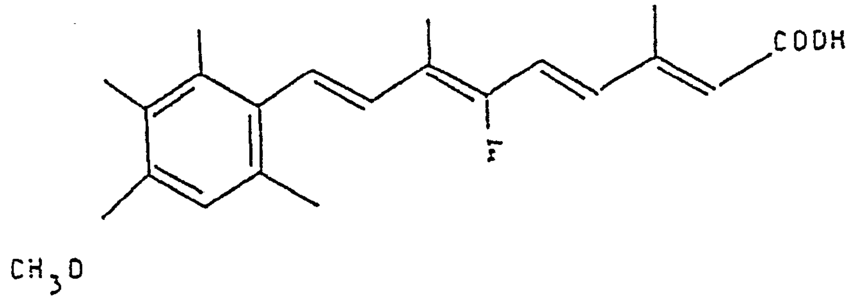

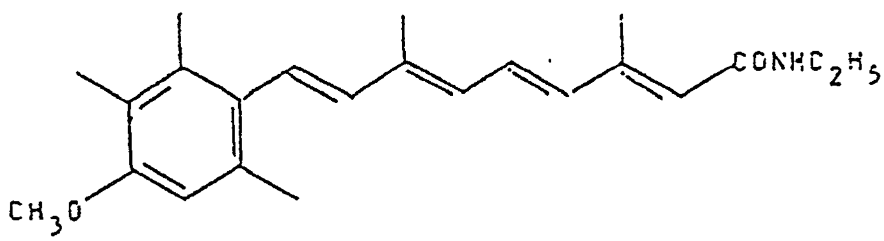

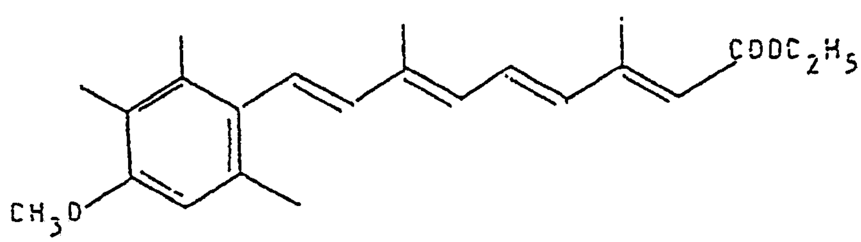

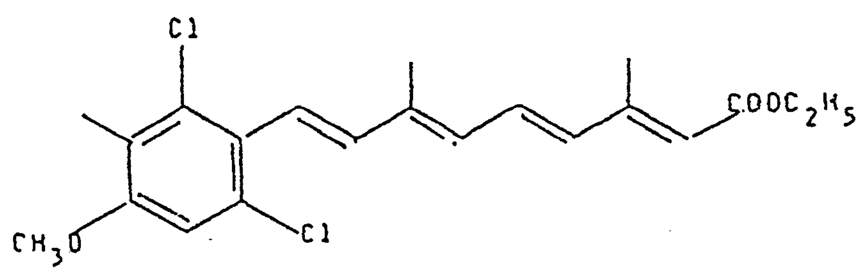

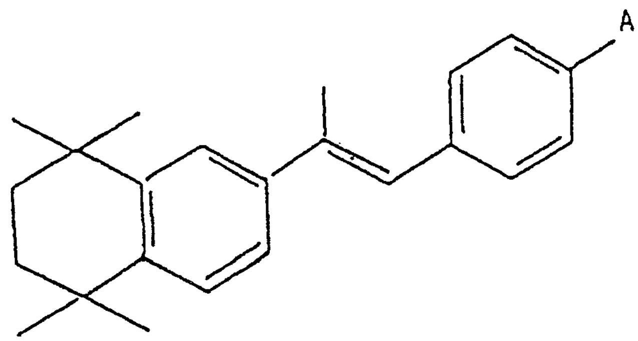

A having the meaning
- $COOC_2H_5$
- $CH_2\ OCH_3$

The 6-amino 4-(substituted amino) 1,2-dihydro 1-hydroxy 2-imino pyrimidine compound is represented by formula

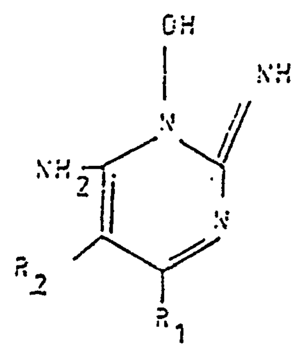

wherein $R_1$ is a moiety selected from moieties of the formula

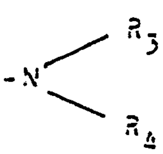

wherein $R_3$ and $R_4$ are selected from hydrogen, lower alkyl, lower alkenyl, lower aralkyl, and lower cycloalkyl, and taken together $R_3$ and $R_4$ may be a heterocyclic moiety selected from aziridinyl, azetidinyl, pyrrolidinyl, piperidino, hexahydroazepinyl, heptamethylenimino, octamethylenimino, morpholino, and 4-(lower-alkyl)piperazinyl, each of said hetero-cyclic moieties having attached as substituents on the carbon atoms 0-3 lower alkyl groups, hydroxy or alkoxy;
wherein $R_2$ is selected from hydrogen, lower alkyl, lower alkenyl, lower alkoxyalkyl, lower cycloalkyl, lower aryl, lower arylalkyl, lower alkylaryle, lower alkylarylalkyl, lower alkoxyarylalkyl, and lower haloarylalkyl;
and the pharmacologically acceptable acid addition salts thereof.

As used herein, the term minoxidil (generic name) denote the following chemical compound and its active derivatives: 2,4,-diamino-6-piperidino-pyrimidine-3-oxide, as in U.S. patents #3,461,461 and #4,139,619.

The pharmaceutical or cosmetic or veterinary preparation of the present invention can be prepared by the conventional techniques for the preparation of lotions, creams, conditioners or shampoos for the scalp or veterinary preparation for pelts. Included also are preparations which can be administered orally.

In addition to the active retinoids and minoxidil of this invention, the various preparations can contain any conventional pharmaceutically acceptable or cosmetically acceptable inert or pharmacodynamically active additives. For example the lotions may be prepared using various forms of alcohols or other solubilizers such as glycols, or esters. The conditioners may contain the normally acceptable commercially

produced compounds such as cetyl alcohol, ceteareth-5,-20 hydantoins, hydrolyzed animal protein, glycol stearate, amodimethicone, paraffin, mineral oil, etc. The topical compositions may also contain various oils including essential fatty acids such as vitamins, steroid hormones including progesterone, estradiols, thyroid hormones, as well as analogues, and certain prostaglandins and prostaglandin analogues.

The topically applied lotions, creams, and conditioners etc. will vary according to the standard art with regard to the amounts of other hydrophilic and hydrophobic contained ingredients including emulsifiers, so that either an oily, semi-oily or oil free product may be obtained. The shampoos may contain any of the conventionally used detergents or soaps and any other compounds used by those familiar with the art. Oil based shampoos are included in these formulations.

The oral preparations may be tablets, liquids and capsules. The pharmaceutically acceptable substances commonly used as preservatives, stabilizers, moisture retainers, emulsifiers, etc. can be present in these preparations. Conventionally acceptable antioxidants such as tocopherols, N-methyl α-tocopheramine, butylated hydroxyanisole and butylated hyroxytoluene, can be also incorporated in the preparations described in this invention.

The dosages in which the retinoids and minoxidil are administered can be varied with the route of administration and the requirements of the subjects.

The retinoid is present in the composition in an amount between of 0.0001 and 5% by weight.

The topical compositions may consist of lotions, creams, conditioners, shampoos, oil compositions, etc. with from 0.001 to 2% weight of all-trans retinoic acid or derivatives, or other retinoids, as the preferred dosages in the described compositions and with dosages of from .01% to 30% minoxidil in suitable vehicles for percutaneous adsorption.

The oral pharmaceutical preparations may be administered at a daily dose of from 0.25 mg to 2 mg per kilogram of body weight for retinoids and from 0.01 to 10.0 mg/kg body weight for minoxidil.

In order to examine the specific action of the retinoids and minoxidil in increasing the rate of hair growth, several types of experiments were performed. The microcapillary method was used in each case to measure the rate of hair growth. Other methods of hair growth measurements using dye staining were also undertaken. In addition, the method of Ebling et al, Journal of Investigative Dermatology, November 1981, was used to study the conversion of vellus to terminal hair by microscopy.

Table I describes the results of studies using male and female subjects. The all-trans retinoic acid and minoxidil in lotion form was applied topically as described in the table and hair growth rates were assessed along with transformation from vellus to terminal hairs. (See separate pages for TABLES).

In fur bearing animals, the rate of fur growth, length of hair, thickness of hair and molting season are controlled by many factors including season, light (wavelength) periodicity, temperature, hormonal factors and nutrition. Controlling all of these variables is impossible, however, animals were selected and areas over the hind quarters were shave in 2 inch diameter circular areas. In some of the animals the areas were treated topically with all-trans retinoic acid and in other animals the retinoid was administered orally in animal chow. Some of the animals served as their own controls, using treated and non-treated areas.

In fur bearing animals, the guard hairs and the pile hairs differ in thickness, length and growth rate. In the rabbits studied, the guard hairs averaged 34 mm and the pile hairs 30 mm in length. The effect of topical application of all-trans retinoic acid was to increase the rate of new hair growth. An effect on the non-shaved fur bearing areas treated with topical all-trans retinoic acid in lotion form, was a decrease in the shedding or molting of fur. The mean rate of hair (fur) growth from treated shaved areas was 0.3 mm/day for 3 rabbits (mean) while in non-treatment shaved areas it averaged 0.2 mm/day (mean of 3 rabbits).

The effect could also be demonstrated in domestic cats and dogs; the same type of experimental procedures were used. The most striking effect in long haired dogs and cats was the retardation of molting or hair shedding. Long haired dogs and cats tended to retain more hair in the anagen phase and there was approximately 50% less shedding during the treatment periods. Both methods of administration were satisfactory. Either topical lotion or cream treatment or systemic treatment by inclusion in animal chow was satisfactory. The daily dosage for animals was 20 mg per kilogram per day in chow or 40 to 15 mg applied topically.

Commercially important fur bearing animals were also used for experimentation. Two male minks were closely clipped over the back hind quarters. The animals were treated on one hind quarter and the other was used as the control. The capillary method for measuring hair growth was used for these studies. The animals were treated by two different methods. The animals were either fed the retinoid in their chow or they were administered the retinoid topically. The daily dose was 20 mg per kg body weight per day in animal chow or 5 mg per day applied topically. The results of these experiments showed that the rate of growth of new pelt was increased approximately 30% by the retinoid treatment.

Experiments using birds (canaries and parakeets) showed that inclusion of the all-trans retinoic acid or

the ethyl ester of all-trans retinoic acid in bird food at a dosage of 30 mg per kilogram bird weight per day retarded the molting process.

The following Examples illustrate the present invention The methods of administration may vary by lotion, cream ointment, pill, supplement to chow and coating for seeds, These Examples are only meant to be illustrative and do not limit the mode of administration nor the ingredients which can be admixed to the present invention, nor the amounts which may be used.

Example I

| Lotion formulation for the topical administration | % wt to wt |
|---|---|
| Active ingredients: | |
| I. All-trans retinoic acid | 0.1 |
| II. Minoxidil: | 3.0 |
| Ethanol | q.s. to 100.0 |
| Propylene glycol | 5.0 |
| Butylated hydroxytoluene | 0.1 |
| Safflower oil | 1.0 |
| α-tocopherol acetate | 0.5 |
| Stabilizer | 0.1 |

Example II

Cream conditioner for Topical Administration

| | |
|---|---|
| Active ingredients: | |
| I. All-trans retinoic acid | 1.0 |
| II. Minoxidil | 10.0 |
| Distilled water | q.s. to 100.0 |
| Cetrimonium Chloride | 5.0 |
| Cetyl alcohol | 4.0 |
| Ethanol | 4.0 |
| Butylated hydroxytoluene | 1.0 |
| Hydrolized animal protein | 0.5 |
| Methylparaben, propylparaben | 0.1 |
| Stabilizer | 0.1 |

Example III

All-trans retinoic acid 0.1 gram and 10 grams of minoxidil are dissolved in 100 ml of acetone, and the solution admixed with 900 g of USP grade hydrophilic ointment to a uniform consistency; one gram of butylated hydroxytoluene is added. The water washable cream ointment thus prepared consists of 0.1% retinoic acid and 10% minoxidil

## Example IV

Tablets for oral administration

| | |
|---|---|
| Minoxidil | 10 mg. |
| Active ingredients all-trans retinoic acid | 25 mg. |
| Lactose | 52 mg. |
| Cornstarch | 20 mg. |
| Microcrystalline cellulose | 40 mg. |
| Talc | 2.5 mg. |
| Magnesium stearate | 0.5 mg. |

The active ingredients are mixed with lactose and granulated using a corn starch paste. The remainder of the above adjuvants was then admixed therein and the mass was tableted. The tablets were then coated with a water-soluble or water-swellable lacquer.

The same formulation can also be used and gelatin can be added to make beadlets. These beadlets are then coated with a lacquer. The beadlets and animal chow can be mixed to the desired dosage level.

The above formulation can also be used in the powdered form for mixing with bird seed and the bird seed can then be sprayed with lacquer.

Liquids, syrups or other formulations can be made consistent with the pharmaceutical art.

RESULTS USING LOTION CONTAINING ALL-TRANS RETINOIC ACID 0.1% AND MINOXIDIL 3%

TABLE I

| Subject Sex | Subject Age | Dosage Lotion | Form of Dosage | Treatment Time | mm/day Control Rate | mm/day Treatment Rate of Growth | % of conversion from vellus to terminal per sq. cm. during treatment |
|---|---|---|---|---|---|---|---|
| M | 37 | 10 ml/day | Topical lotion applied to Scalp | 2 mths. | 0.23 | 0.30 | 11% |
| M | 62 | " | " | " | 0.21 | 0.29 | 22% |
| M | 38 | " | " | " | 0.35 | 0.42 | 35% |
| F | 43 | " | " | " | 0.37 | 0.39 | 13% |
| F | 38 | " | " | " | 0.31 | 0.35 | 18% |
| F | 64 | " | " | " | 0.24 | 0.29 | 17% |

## Claims

1. A composition useful for increasing the rate of growth of hair, and for treating various type of alopecias comprising a retinoid and a 6-amino 4-(substituted amino) 1,2-dihydro 1-hydroxy 2-imino pyrimidine compound of formula :

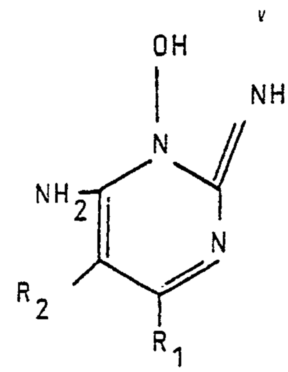

wherein $R_1$ is a moiety selected from moieties of the formula

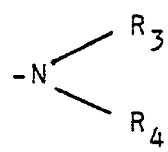

wherein $R_3$ and $R_4$ are selected from hydrogen, lower alkyl, lower alkenyl, lower aralkyl, and lower cycloalkyl, and taken together $R_3$ and $R_4$ may be a heterocyclic moiety selected from aziridinyl, azetidinyl, pyrrolidinyl, piperidino, hexahydroazepinyl, heptamethylenimino, octamethylenimino, morpholino, and 4-(lower-alkyl)piperazinyl, each of said hetero cyclic moieties having attached as substituents on the carbon atoms 0-3 lower alkyl groups, hydroxy or alkoxy wherein $R_2$ is selected from hydrogen, lower alkyl, lower alkenyl, lower alkoxyalkyl, lower cycloalkyl, lower aryl, lower arylalkyl, lower alkylaryle lower alkylarylalkyl, lower alkoxyarylalkyl, and lower haloarylalkyl and the pharmacologically acceptable acid addition salts thereof, in an amount producing a synergistic effect.

2. Composition according to claim 1 characterized in that the compound of formula I is 2,4-diamino 6-piperidino pyrimidine 3-oxide or Minoxidil.

3. Composition according to claim 1 or 2 characterized in that the retinoid is an ester, an ether or a salt of a retinoid acid.

4. Composition according to claim 1 or 2 characterized in that the retinoid is a stereoisomer of vitamin A acid or vitamin $A_2$ acid, 5,6-epoxy vitamin A acid, dehydro vitamin A acid, anhydro vitamin A acid or the aldehyde, alcohols, esters, ethers thereof.

5. Composition according to claim 1 or 2,
characterized in that the retinoid is a naturally occuring metabolite of vitamin A or vitamin A acid.

6. Composition according to claim 5, characterized in that the metabolite is 4-hydroxy retinoic acid, 4-keto retinoic acid, 4-oxo retinoic acid, 5,8-oxy retinoic acid or stereoisomers thereof and aldehydes, esters thereof.

7. Composition according to claim 1 or 2,
characterized in that the retinoid is a compound of formula :

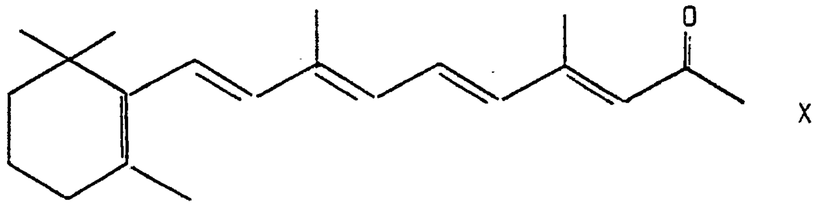

wherein X has the meanings
$-OCH_2CONH_2$; mixed $-OCH_2CH(OH)CH_3$ and $-OCH(CH_3)CH_2OH$ ;$-OCH_2\ CH_2\ OH$ ;

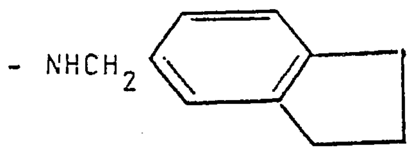

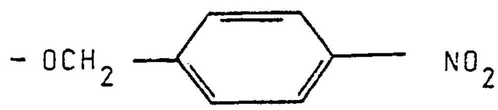

$-OCH_2C_6H_5$, or 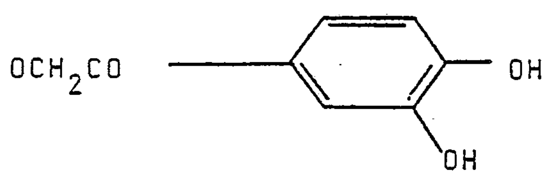

8. Composition according to claim 1 or 2 characterized in that the retinoid is all trans retinoic acid; all-trans retinoic acetate, retinaldehyde, 13-cis retinoic acid, retinoic acid ethylamide, retinoic acid 2-hydroxyethyl amide, retinoic acid p-hydroxyphenylamide, axerophthene, retinal, retinol, retinyl methylether, β-all-trans retinoic acid, 7,8-dehydro analog of retinoic acid, 5,6-epoxy analog of retinoic acid, retinyl methylthioether, 4-oxo analog of retinoic acid, N-methyl dimethyl dioxolan retinamide, retinyl n-butyl ether, N-(O-carboxyphenyl) retinamide, retinyl tert-butyl ether, retinylacetate, N-(p-carboxyphenyl) retinamide, N-benzoyl retinylamide, retinyl palmitate, retinylidene ethylcyano acetate.

9. Composition according to claim 1 or 2,
characterized in that the retinoid coresponds to compound of formula :

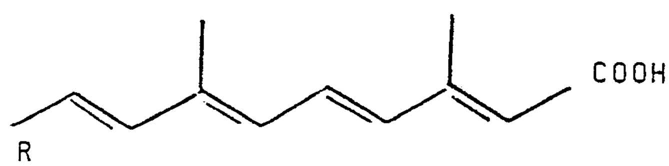

R having the meanings :

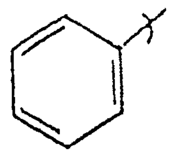

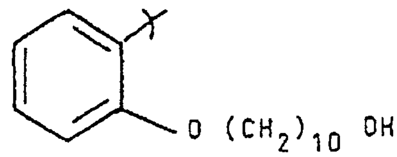

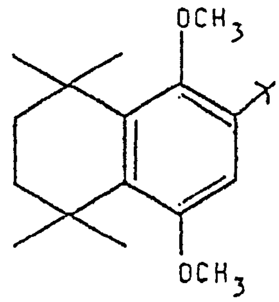

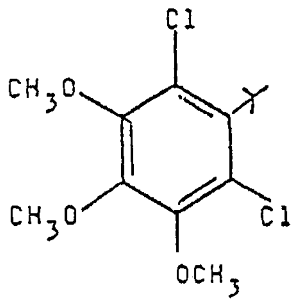

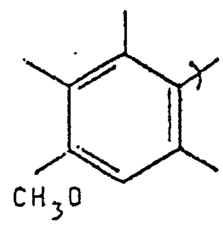

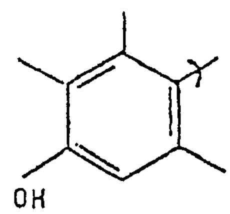

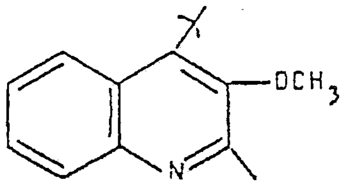

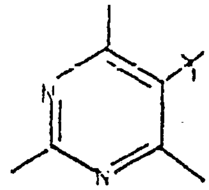

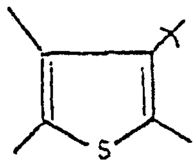

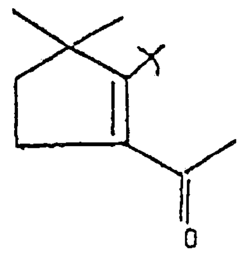

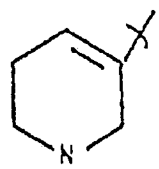

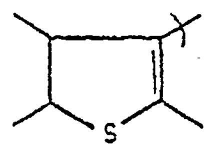

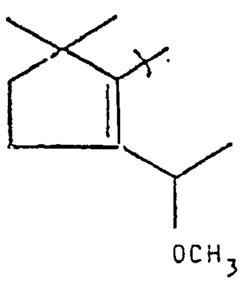

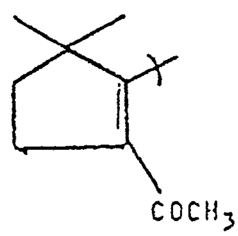

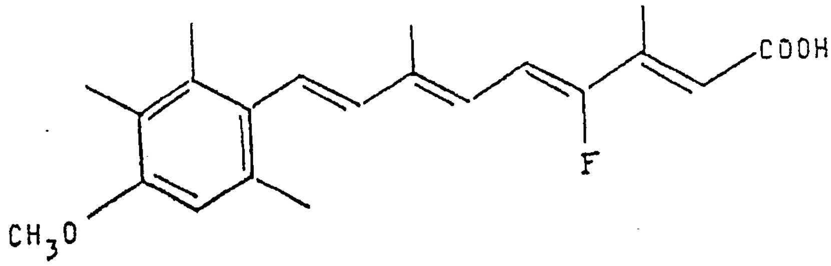

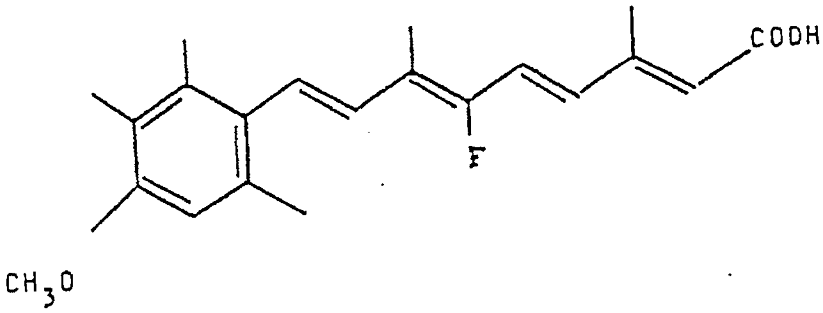

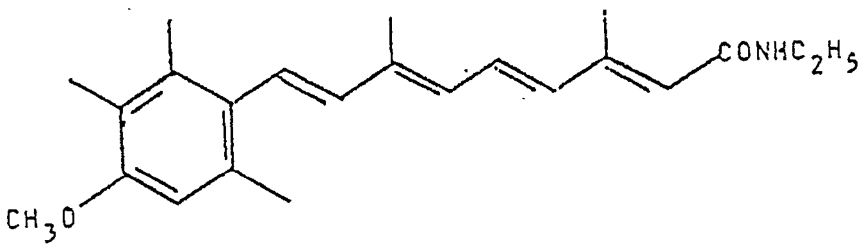

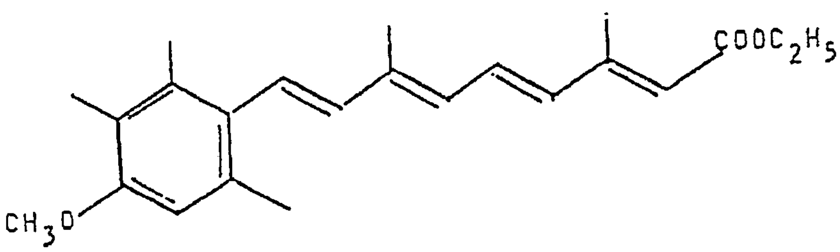

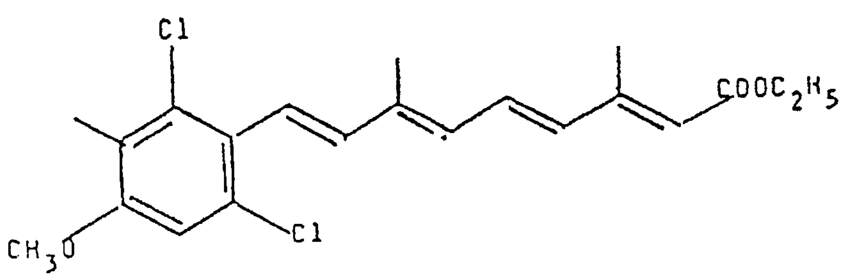

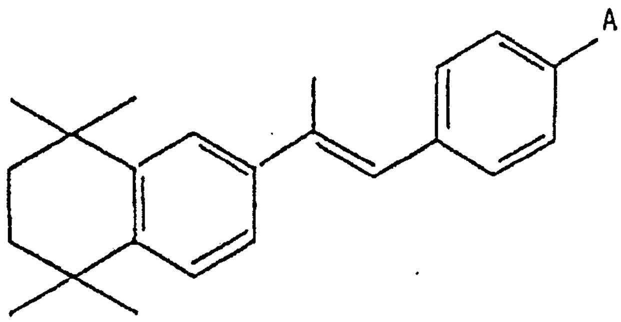

A having the meaning $-COOC_2H_5$
$- CH_2 OCH_3$

10. Composition as defined in anyone of claims 1 to 9 characterized in that the composition is under the form of a topical lotion, cream, ointment, conditionner or shampoo.

11. Composition according to anyone of claims 1 to 10, characterized in that the retinoid is present in an amount of between 0.0001 and 5% by weight.

12. Composition according to anyone of claims 1 to 11, characterized in that the compound of formula I is present in an amount of between 0.01 and 30% by weight.

13. Composition as defined in any one of claims 1 to 12 wherein the retinoid is all trans retinoic acid and the compound of formula I is Minoxidil.

14. Composition as defined in anyone of claims 1 to 13 further containing a pharmaceutically or cosmetically acceptable or pharmacodynamically active additive.

15. Composition as defined in anyone of claims 1 to 14 characterized in that it contains as solubilizers a mixture of ethanol and propyleneglycol.

16. Composition as defined in anyone of claim 1 to 15 for its use in the therapeutic treatment of various types of alopecias in mammals.

17. Composition as defined in anyone of claims 1 to 15 for its use in the cosmetic treatment of hair.

18. Process for the cosmetic treatment of hair characterized in that a composition as defined in anyone of claims 1 to 15 is used.

19. Use of the composition as defined in anyone of claims 1 to 15 for the preparation of a pharmaceutical composition for the treatment of alopecia.

## Revendications

1. Composition utile pour augmenter la vitesse de pousse des cheveux et pour le traitement de divers types d'alopécie, comprenant un rétinoïde et un composé 6-amino-4-(amino-substitué)-1,2-dihydro-1-hydroxy-2-imino-pyrimidine, de formule :

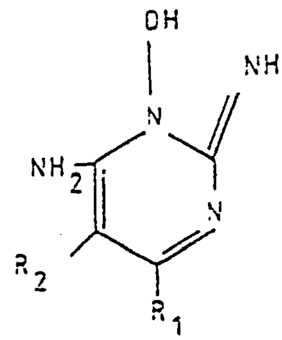

dans laquelle $R_1$ est un groupe choisi parmi les groupements de formule

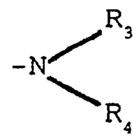

dans laquelle $R_3$ et $R_4$ sont choisis parmi des atomes d'hydrogène et les groupes alkyle inférieurs, alcényle inférieurs, aralkyle inférieurs et cycloalkyle inférieurs, ou bien, pris conjointement, $R_3$ et $R_4$ peuvent représenter un groupement hétérocyclique choisi parmi les groupes aziridinyle, azétidinyle, pyrrolidinyle, pipéridino, hexahydroazépinyle, heptaméthylène-imino, octaméthylène-imino, morpholino et 4-(alkyl inférieur)-pipérazinyle, chacun de ces groupements hétérocycliques portant, en tant que substituants sur les atomes de carbone, de 0 à 3 groupes alkyle inférieurs, hydroxy ou alcoxy, et dans laquelle $R_2$ est choisi parmi un atome d'hydrogène et les groupes alkyle inférieurs, alcényle inférieurs, alcoxyalkyle inférieurs, cycloalkyle inférieurs, aryle inférieurs, arylalkyle inférieurs, alkylaryle inférieurs, alkylarylalkyle inférieurs, alcoxyarylalkyle inférieurs et halogénoarylalkyle inférieurs, ou un sel d'addition d'acide pharmacologiquement acceptable de ce composé, en une quantité donnant un effet de synergie.

2. Composition conforme à la revendication 1, caractérisée en ce que le composé de formule I est le 2,4-diamino-6-pipéridino-pyrimidine-3-oxyde, ou minoxidil.

3. Composition conforme à la revendication 1 ou 2, caractérisée en ce que le rétinoïde est un ester, un éther ou un sel d'un acide rétinoïde.

4. Composition conforme à la revendication 1 ou 2, caractérisée en ce que le rétinoïde est un stéréo-isomère de l'acide de la vitamine A ou de l'acide de la vitamine $A_2$, de l'acide de la vitamine A 5,6-époxydé, de l'acide de la déhydrovitamine A, de l'acide de l'anhydrovitamine A, ou des aldéhydes, alcools, esters ou éthers correspondants.

5. Composition conforme à la revendication 1 ou 2, caractérisée en ce que le rétinoïde est un métabolite naturel de la vitamine A ou de l'acide de la vitamine A.

6. Composition conforme à la revendication 5, caractérisée en ce que le métabolite est l'acide 4-hydroxy-rétinoïque, l'acide 4-cétorétinoïque, l'acide 4-oxo-rétinoïque, l'acide 5,8-oxyrétinoïque, ou bien l'un de leurs stéréo-isomères, ou bien l'un des aldéhydes et esters correspondants.

7. Composition conforme à la revendication 1 ou 2, caractérisée en ce que le rétinoïde est un composé de formule :

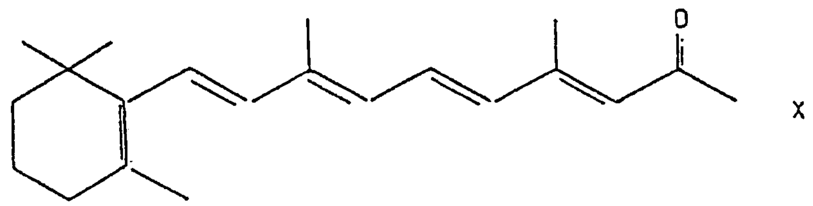

dans laquelle X représente :
$-OCH_2CONH_2$ ; $-OCH_2CH(OH)CH_3$ et $-OCH(CH_3)CH_2OH$ (mélange) ;
$-OCH_2-CH_2OH$ ;

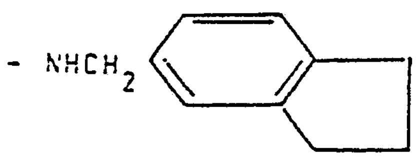

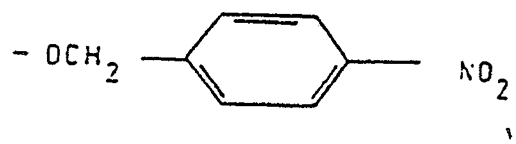

$-OCH_2C_6H_5$ ; ou $OCH_2CO$ 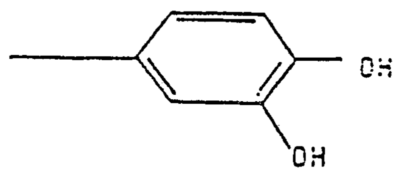

8. Composition conforme à la revendication 1 ou 2, caractérisée en ce que le rétinoïde est l'acide tout-trans-rétinoïque, l'acétate tout-trans-rétinoïque, le rétinaldéhyde, l'acide 13-cis-rétinoïque, l'éthylamide de l'acide rétinoïque, le 2-hydroxyéthylamide de l'acide rétinoïque, le p-hydroxyphénylamide de l'acide rétinoïque, l'axérophtène, le rétinal, le rétinol, le rétinyl-méthyl-éther, l'acide $\beta$-tout-trans-rétinoïque, l'analogue 7,8-déhydro de l'acide rétinoïque, l'analogue 5,6-époxy de l'acide rétinoïque, le rétinyl-méthyl-thio-éther, l'analogue 4-oxo de l'acide rétinoïque, le N-méthyl-(diméthyl-dioxolanyl)-rétinamide, le rétinyl-n-butyl-éther, le N-(o-carboxyphényl)-rétinamide, le rétinyl-t-butyl-éther, l'acétate de rétinyle, le N-(p-carboxyphényl)-rétinamide, la N-benzoyl-rétinylamine, le palmitate de rétinyle, ou le cyanoacétate de rétinylidène-éthyle.

9. Composition conforme à la revendication 1 ou 2, caractérisée en ce que le rétinoïde correspond à un composé de formule

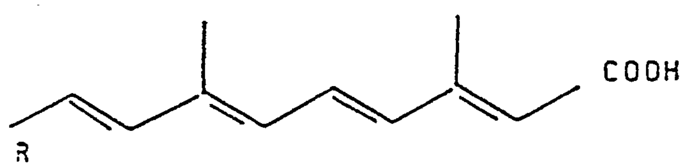

dans laquelle R représente :

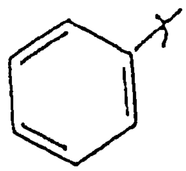

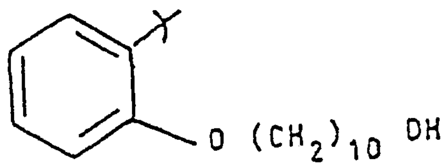

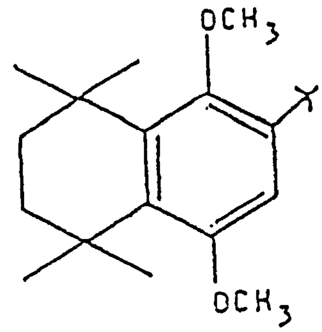

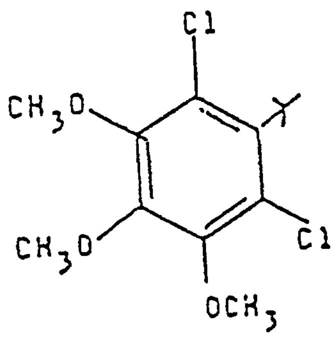

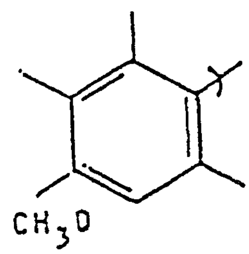

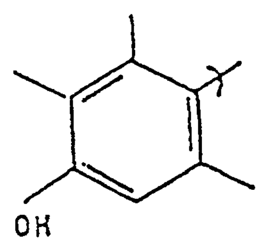

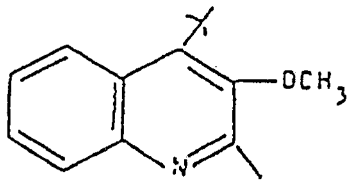

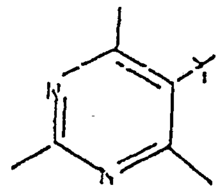

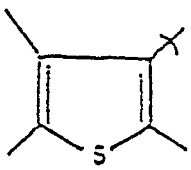

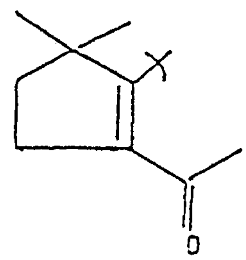

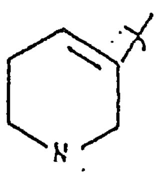

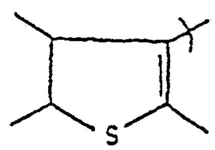

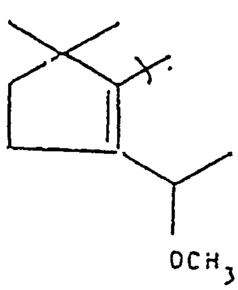

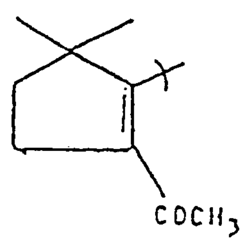

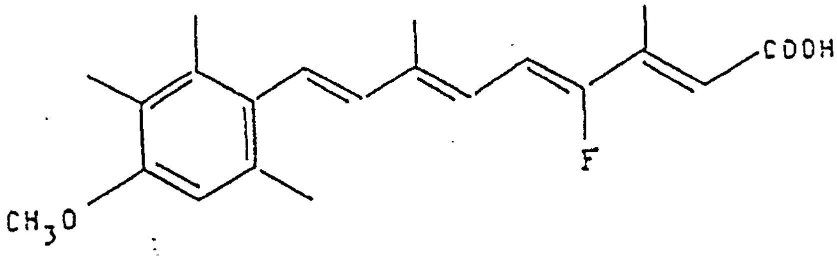

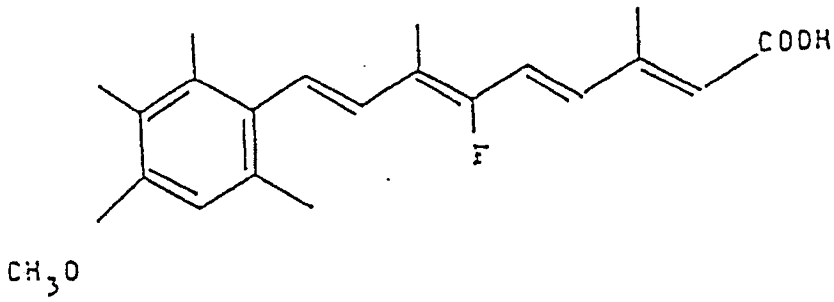

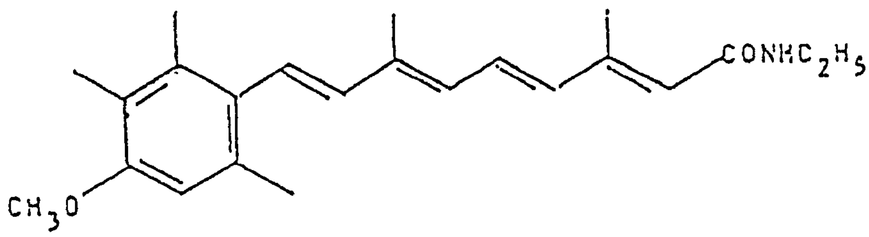

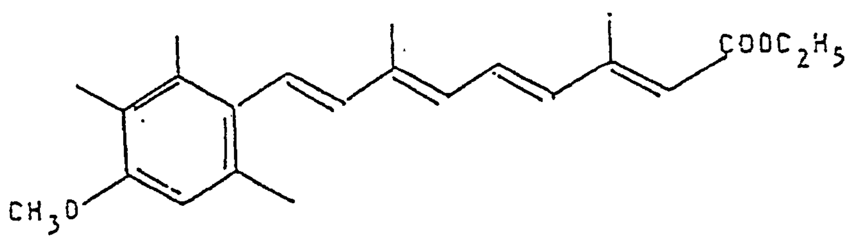

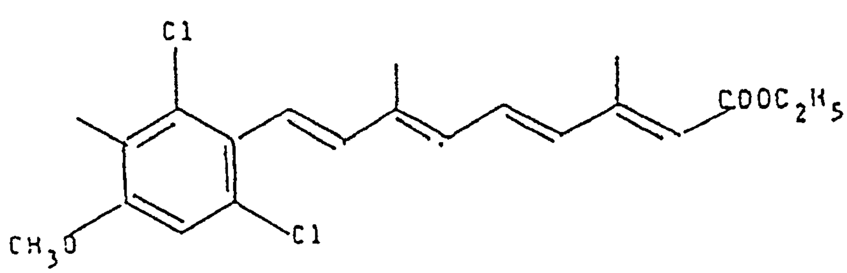

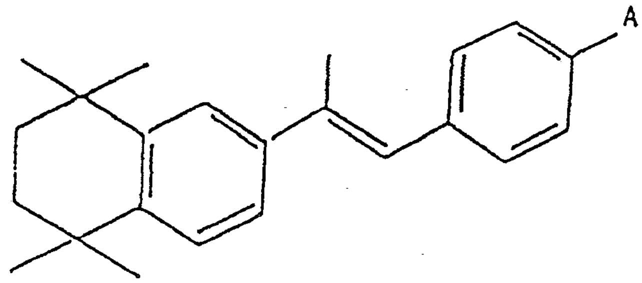

A représentant $-COOC_2H_5$ ou $-CH_2OCH_3$.

**10.** Composition telle que définie dans l'une quelconque des revendications 1 à 9, caractérisée en ce que la composition se trouve sous la forme d'une lotion, crème, pommade, agent de conditionnement ou shampooing, pour application topique.

**11.** Composition conforme à l'une quelconque des revendications 1 à 10, caractérisée en ce que le rétinoïde s'y trouve en une proportion comprise entre 0,0001 et 5 % en poids.

**12.** Composition conforme à l'une quelconque des revendications 1 à 11, caractérisée en ce que le composé de formule I s'y trouve en une proportion comprise entre 0,01 et 30 % en poids.

**13.** Composition telle que définie dans l'une quelconque des revendications 1 à 12, dans laquelle le rétinoïde est l'acide tout-trans-rétinoïque et le composé de formule I est le minoxidil.

**14.** Composition telle que définie dans l'une quelconque des revendications 1 à 13, contenant en outre un adjuvant acceptable du point de vue pharmaceutique ou cosmétique, ou qui présente une activité pharmacodynamique.

**15.** Composition telle que définie dans l'une quelconque des revendications 1 à 14, caractérisée en ce qu'elle contient, comme solubilisant, un mélange d'éthanol et de propylèneglycol.

**16.** Composition telle que définie dans l'une quelconque des revendications 1 à 15, destinée à être utilisée dans le traitement thérapeutique de divers types d'alopécie chez les mammifères.

**17.** Composition telle que définie dans l'une quelconque des revendications 1 à 15, destinée à être utilisée dans un traitement cosmétique des cheveux.

**18.** Procédé pour le traitement cosmétique des cheveux, caractérisé en ce qu'on utilise une composition telle que définie dans l'une quelconque des revendications 1 à 15.

**19.** Utilisation de la composition telle que définie dans l'une quelconque des revendications 1 à 15 pour la préparation d'une composition pharmaceutique destinée au traitement de l'alopécie.

## Ansprüche

**1.** Zusammensetzung, die zur Erhöhung der Haarwachstumsrate und zur Behandlung verschiedener Arten von Alopecia nützlich ist, umfassend ein Retinoid und eine 6-Amino-4-(substituiertes Amino)-1,2-dihydro-1-hydroxy-2-iminopyrimidin-Verbindung der Formel enthält

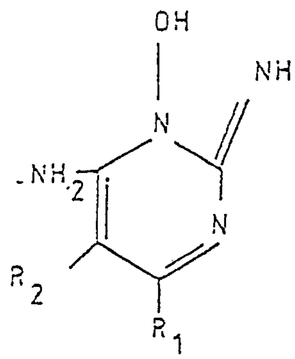

worin $R_1$ einen Anteil darstellt, ausgewählt aus Anteilen der Formel

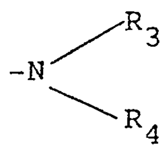

worin $R_3$ und $R_4$ ausgewählt sind aus Wasserstoff, Niedrigalkyl, Niedrigalkenyl, Niedrigaralkyl und Niedrigcycloalkyl, und wenn $R_3$ und $R_4$ zusammengenommen sind, können sie ein heterozyklischer Anteil sein, der ausgewählt ist aus Aziridinyl, Azetidinyl, Pyrrolidinyl, Piperidino, Hexahydroazepinyl, Heptamethylenimino, Octamethylenimino, Morpholino und 4-(Niedrigalkyl)piperazinyl, wobei jeder der heterozyklischen Anteile an den Kohlenstoffatomen 0 bis 3 Niedrigalkylgruppen, Hydroxy oder Alkoxy als Substituenten gebunden aufweisen; worin $R_2$ ausgewählt ist aus Wasserstoff, Niedrigalkyl, Niedrigalkenyl, Niedrigalkoxyalkyl, Niedrigcycloalkyl, Niedrigaryl, Niedrigarylalkyl, Niedrigalkylaryl, Niedrigalkylarylalkyl, Niedrigalkoxyarylalkyl und Niedrighaloarlalkyl; und die pharmakologisch akzeptablen Säureadditionssalze davon, in einer Menge, die einen synergistischen Effekt erzeugt.

2. Zusammensetzung nach Anspruch 1, dadurch **gekennzeichnet**, dass die Verbindung der Formel (I) 2,4-Diamino-6-piperidinopyrimidin-3-oxid oder Minoxidil ist.

3. Zusammensetzung nach Anspruch 1 oder 2, dadurch **gekennzeichnet**, dass das Retinoid ein Ester, ein Ether oder ein Salz von einer Retinoesäure ist.

4. Zusammensetzung nach Anspruch 1 oder 2, dadurch **gekennzeichnet**, dass das Retinoid ein Stereoisomer von Vitamin A-Säure oder von Vitamin $A_2$-Säure, 5,6-Epoxy-Vitamin A-Säure, Dehydro-Vitamin A-Säure, Anhydro-Vitamin A-Säure oder dem Aldehyd, Alkoholen, Estern oder Ethern davon ist.

5. Zusammensetzung nach Anspruch 1 oder 2, dadurch **gekennzeichnet**, dass das Retinoid ein natürlich auftretender Metabolit von Vitamin A oder Vitamin A-Säure ist.

6. Zusammensetzung nach Anspruch 5, dadurch **gekennzeichnet**, dass der Metabolit 4-Hydroxyretinoesäure, 4-Ketoretinoesäure, 4-Oxoretinoesäure, 5,8-Oxyretinoesäure oder Stereoisomere davon und Aldehyde, Ester davon ist.

7. Zusammensetzung nach Anspruch 1 oder 2, dadurch **gekennzeichnet**, dass das Retinoid eine Verbindung der Formel ist:

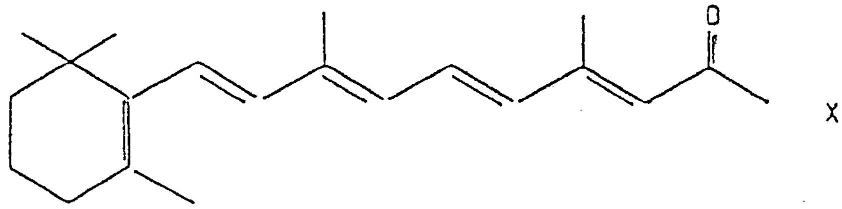

worin X die folgenden Bedeutungen aufweist:
$-OCH_2CONH_2$; gemischtes $-OCH_2CH(OH)CH_3$ und $-OCH(CH_3)CH_2OH$; $-OCH_2CH_2OH$;

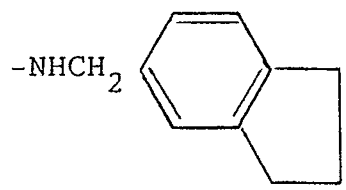

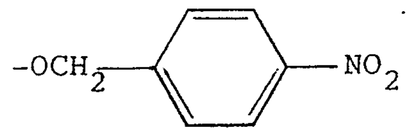

$-OCH_2C_6H_5$ oder 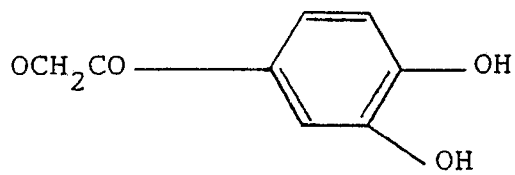

8. Zusammensetzung nach Anspruch 1 oder 2, dadurch **gekennzeichnet**, dass das Retinoid all-trans-Retinoesäure, all-trans-Retinoeacetat, Retinaldehyd, 13-cis-Retinoesäure, Retinoesäureethylamid, Retinoesäure-2-hydroxyethylamid, Retinoesäure-p-hydroxyphenylamid, Axerophthen, Retinal, Retinol, Retinylmethylether, β-all-trans-Retinoesäure, 7,8-Dehydroanalogon von Retinoesäure, 5,6-Epoxyanalogon von Retinoesäure, Retinylmethylthioether, 4-Oxoanalogon von Retinoesäure, N-Methyldimethyldioxolan-retinamid, Retinyl-n-butylether, N-(O-Carboxyphenyl)retinamid, Retinyl-tert-butylether, Retinylacetat, N-(p-Carboxyphenyl)retinamid, N-Benzoylretinylamid, Retinylpalmitat, Retinylidenethylcyanoacetat ist.

9. Zusammensetzung nach Anspruch 1 oder 2, dadurch **gekennzeichnet**, dass das Retinoid einer Verbindung der Formel entspricht:

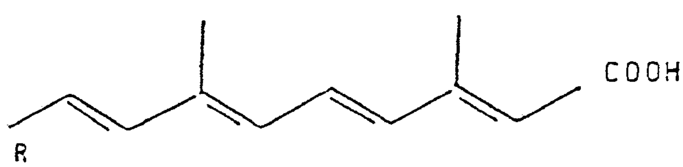

worin R die folgenden Bedeutungen aufweist:

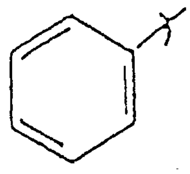

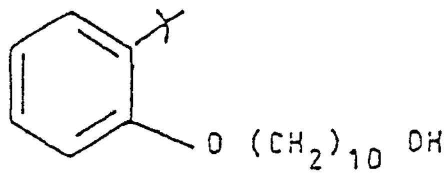

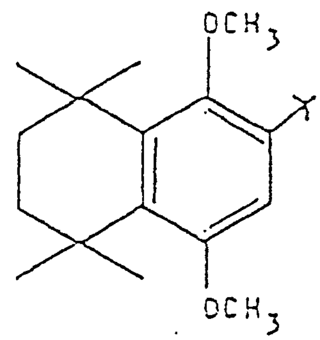

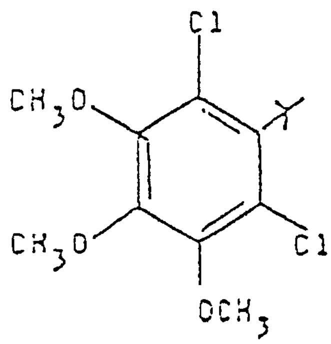

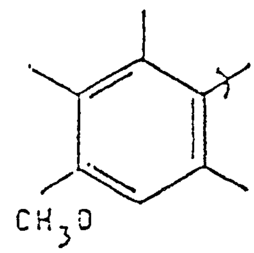

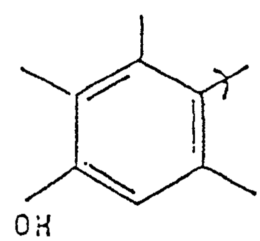

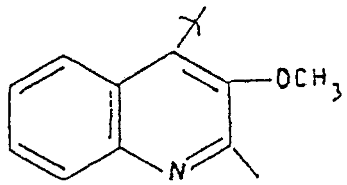

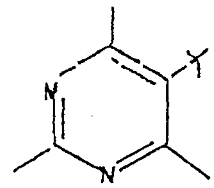

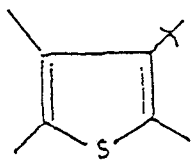

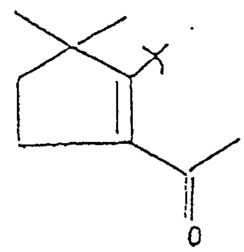

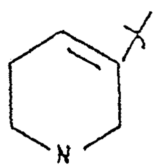

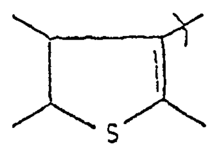

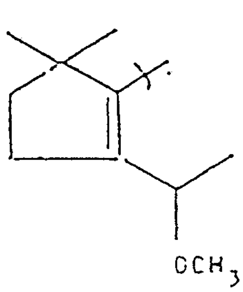

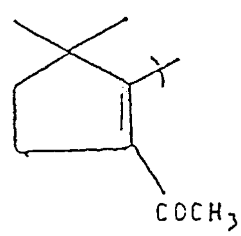

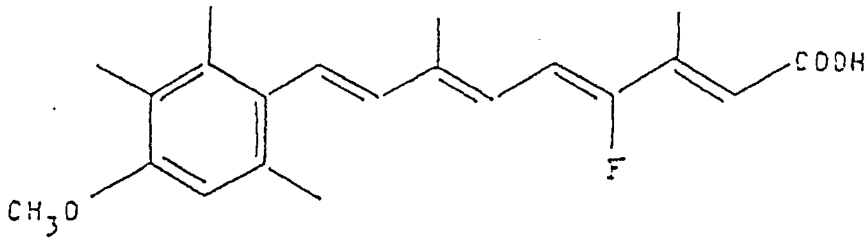

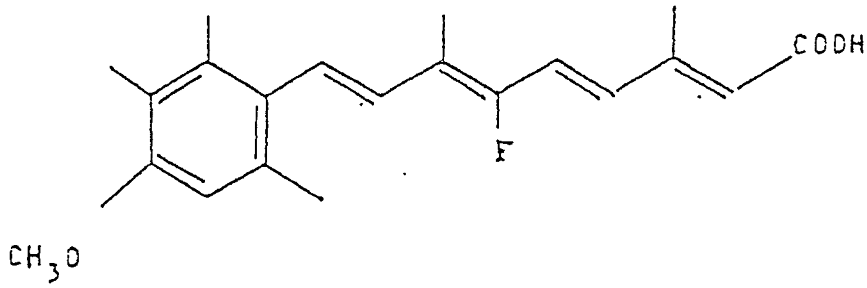

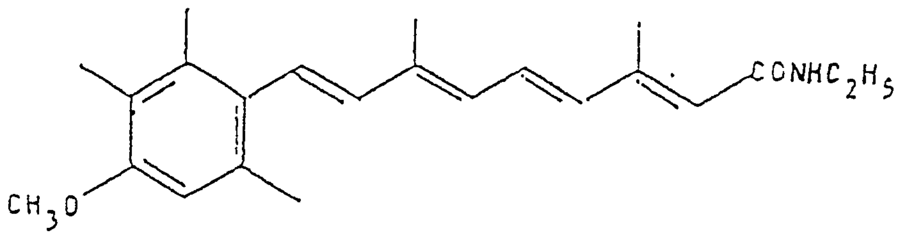

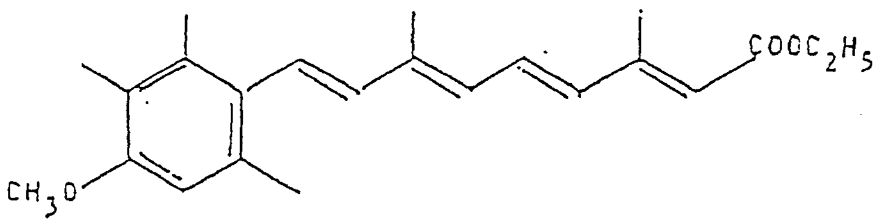

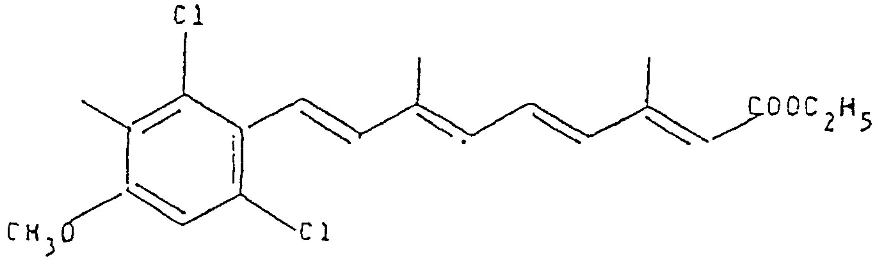

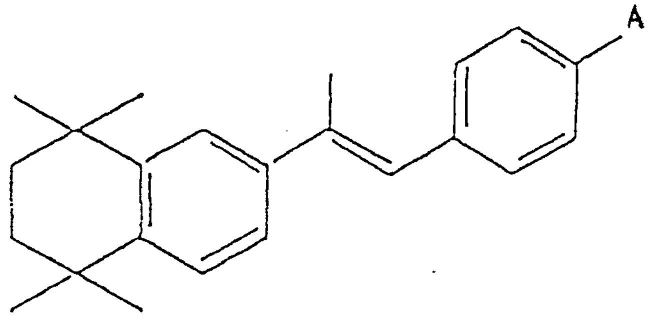

und worin A die Bedeutung $-COOC_2H_5$, $-CH_2OCH_3$ aufweist.

10. Zusammensetzung nach einem der Ansprüche 1 bis 9, dadurch **gekennzeichnet**, dass die Zusammensetzung in Form einer topischen Lotion, Creme, Salbe, Haarpflegemittel oder Shampoo vorliegt.

11. Zusammensetzung nach einem der Ansprüche 1 bis 10, dadurch **gekennzeichnet**, dass das Retinoid in einer Menge von zwischen 0,0001 und 5 Gew.% vorhanden ist.

12. Zusammensetzung nach einem der Ansprüche 1 bis 11, dadurch **gekennzeichnet**, dass die Verbindung der Formel (I) in einer Menge von zwischen 0,01 und 30 Gew.% vorhanden ist.

13. Zusammensetzung nach einem der Ansprüche 1 bis 12, dadurch **gekennzeichnet**, dass das Retinoid all-trans-Retinoesäure und die Verbindung der Formel (I) Minoxidil ist.

14. Zusammensetzung nach einem der Ansprüche 1 bis 13, dadurch **gekennzeichnet**, dass sie weiterhin ein pharmazeutisch oder kosmetisch akzeptables oder pharmakodynamisch aktives Additiv enthält.

15. Zusammensetzung nach einem der Ansprüche 1 bis 14, dadurch **gekennzeichnet**, dass sie als Lösungsmittel eine Mischung aus Ethanol und Propylenglykol enthält.

16. Zusammensetzung nach einem der Ansprüche 1 bis 15 für ihre Verwendung in der therapeutischen Behandlung von verschiedenen Arten von Alopecia bei Säugetieren.

17. Zusammensetzung nach einem der Ansprüche 1 bis 15 für ihre Verwendung in der kosmetischen Haarbehandlung.

18. Verfahren für die kosmetische Behandlung von Haar, dadurch **gekennzeichnet**, dass eine Zusammensetzung nach einem der Ansprüche 1 bis 15 verwendet wird.

19. Verwendung der Zusammensetzung nach einem der Ansprüche 1 bis 15 zur Herstellung einer pharmazeutischen Zusammensetzung für die Behandlung von Alopecia.